Europäisches Patentamt

European Patent Office

Office européen des brevets

(11) Publication number: **0 095 089**
**B1**

(12) **EUROPEAN PATENT SPECIFICATION**

(45) Date of publication of patent specification: **05.03.86**

(51) Int. Cl.⁴: **G 01 N 33/542**

(21) Application number: **83104621.4**

(22) Date of filing: **11.05.83**

(54) Improved homogeneous binding assay method and reagent system, test kit and test device therefor.

(30) Priority: **24.05.82 US 381165**

(43) Date of publication of application:
**30.11.83 Bulletin 83/48**

(45) Publication of the grant of the patent:
**05.03.86 Bulletin 86/10**

(84) Designated Contracting States:
**CH DE FR GB IT LI**

(56) References cited:
**EP-A-0 034 775**
**EP-A-0 051 213**
**US-A-3 935 074**
**US-A-4 160 645**
**US-A-4 281 061**

**CLINICAL CHEMISTRY, vol. 27, no. 9,
September 1981, pages 1499-1504, Easton,
Pennsylvania, USA, R.J. TYHACH et al.:
"Adaption of prosthetic-group-label
homogeneous immunoassay to reagent-strip
format"**

(73) Proprietor: **MILES LABORATORIES, INC.**
**1127 Myrtle Street**
**Elkhart Indiana 46514 (US)**

(72) Inventor: **Dean, Kenneth James**
**26921 Dumbarton Court**
**Elkhart, IN 46514 (US)**

(74) Representative: **Jesse, Ralf-Rüdiger, Dr. et al**
**Bayer AG Konzernverwaltung RP**
**Patentabteilung**
**D-5090 Leverkusen 1 Bayerwerk (DE)**

(56) References cited:

**CLINICAL CHEMISTRY, vol. 25, no. 5, May 1979,
pages 686-691, Easton, Pennsylvania, USA,
R.C. WONG et al.: "Substrate-labeled
fluorescent immunoassay for phenytoin in
human serum"**

**BIOCHEMICAL AND BIOPHYSICAL RESEARCH
COMMUNICATIONS, vol. 47, no. 4, 1972, pages
846-851, K.E. RUBENSTEIN et al.:
"Homogeneous enzyme immunoassay. A new
immunochemical technique"**

Courier Press, Leamington Spa, England.

# Description

## Background of the invention
### 1. Field of the invention

The development of specific binding assay techniques has provided extremely useful analytical methods for determining various organic substances of diagnostic, medical, environmental and industrial importance which appear in liquid mediums at very low concentrations. Specific binding assays are based on the interaction between the substance under determination, herein referred to as analyte, and a binding partner thereof. Where one of the analyte and its binding partner is an antibody and the other is a corresponding hapten or antigen, the assay is known as an immunoassay. Other binding interactions between the analyte and a binding partner serve as the basis of binding assays, including the binding interactions between hormones, vitamins, metabolites, and pharmacological agents, and their respective receptors and binding substances.

The first binding assay to be developed was the radioimmunoassay which employs a radioactive isotope as the label. Because of the inconvenience and difficulty of handling radioactive materials, assay systems have been devised using materials other than radioisotopes as the label component, including enzyme cofactors, enzyme substrates, enzyme modulators, e.g., activators and inhibtors, cycling reactants, spin radicals, enzymes, bacteriophages, metals and organometallic complexes, organic and inorganic catalysts, prosthetic groups, chemiluminescent reactants, and fluorescent molecules.

In a binding assay for determing an analyte in a test sample, the sample is combined with a reagent system which includes a labeled conjugate which becomes partitioned between a bound form, the bound-species, and its unbound form, the free-species, depending upon the amount of analyte present. Where the labeled conjugate in the bound-species is essentially indistinguishable in the presence of the labeled conjugate in the free-species by the means used to monitor the label, as in the case of radioimmunoassay, the bound-species and the free-species must be physically separated in order to complete the assay. This type of assay is referred to in the art as "heterogeneous". Where the bound-species and free-species forms of the labeled conjugate can be distinguished in the presence of each other, a "homogeneous" format can be followed and the separation step avoided.

## 2. Description of the prior art

Many different assay formats can be devised for carrying out homogeneous binding assays. Commonly, a competitive binding format is followed wherein a labeled form of the analyte, or of an analog thereof, competes with the analyte from the test sample for binding to a binding partner of the analyte, e.g., antibody. In certain applications, however, a great advantage is offered by a direct binding format wherein a labeled binding partner is used, any analyte in the sample binding directly to the labeled material to cause a measurable change in the label response. Nonetheless, the use of a labeled binding partners in homogeneous binding assay has been very limited. Representative of the art are U.S. Pat. Nos. 4,233,402; 3,996,345; 4,287,300; 4,208,479; and 4,256,834; Litman et al, Anal. Biochem. 106:223(1980); Ullman et al, J. Biol, Chem. 251:4172 (1976); Wei and Rube, Clin. Chem. 23:1386 (1977); and Gibbons et al, Clin. Chem. 27: 1602 (1981).

A particularly unique and advantageous homogeneous binding assay employing a labeled binding partner is described in EP—A—0088974, and assigned to the instant assignee. In one aspect, this assay system employs a single labeled monoclonal antibody reagent. A test sample suspected to contain the analyte of interest is combined with a labeled monoclonal antibody preparation in which antibody to the analyte is substantially the only labeled component. The label employed is selected such that a detectable response is measurably different in a qualitative or quantitative sense when the labeled antibody is bound to the analyte compared to when not so bound. The resulting detectable response is therefore a function of the analyte in the test sample. As the analyte concentration increases, there is increased binding of labeled antibody and thus increased modulation of the detectable response of the label.

U.S. Patent No. 4,281,061 describes a homogeneous immunoassay based on competitive binding between analyte and labeled analyte for a binding partner and which employs anti-(binding partner) for the purpose of enhancing sensitivity. Clin. Chem. 27:1797 (1981) is a general review of the application of monoclonal antibodies in clinical immunology. German OLS 3,006,709 and 3,006,710 describe homogeneous immunoassays based on competitive binding between analyte and labeled analyte for a binding partner. The label employed is an enzyme modulator, e.g., inhibitor, and one of the binding partner, the modulator label or the enzyme that is modulated is sterically enlarged such as by binding of antibody.

## Summary of the invention

The present invention provides an improved homogeneous binding assay method for determining a polyvalent analyte in a test sample, wherein said sample is combined with labeled anti-analyte and a reagent detection system for the label comprised in said labeled anti-analyte, said label interacting with a member of said detection system to provide a response which is measurably different when said labeled anti-analyte is bound to said analyte compared to when not so bound due to steric hindrance of access of said detection system member to said label; and wherein said response it measured as a function of the analyte in the test sample, characterized in that said test sample is combined additionally with a macromolecular binder which binds with said analyte but not significantly with said labeled anti-analyte, whereby the steric hindrance is specifically enhanced. In the reaction medium, the auxiliary binder will become bound to analyte/labeled binding partner complex, increasing the steric bulk of this bound-species of the labeled binding partner and thereby providing increased steric blockage or impairment of the interaction of the label with the reagent detection system. The auxiliary binder may be any substance of significant size which will bind the analyte but not the labeled binding partner. Preferably the auxiliary binder will be a binding protein such as an antibody or a fragment or aggregate thereof. Also, the label preferably is a participant in an enzyme-catalyzed reaction such as an enzyme substrate, a coenzyme, an enzyme prosthetic group, an enzyme inhibitor, or an enzyme itself.

The present improvement is applicable to homogeneous binding assays for polyvalent analytes, which are analytes of substantial size, usually larger than 10,000 daltons in molecular weight, than can accommodate simultaneous binding of the labeled binding partner and the auxiliary binder. A principal advantage of the present invention is that the degree of modulation of the label response upon binding of analyte with the labeled binding partner can be substantially increased by the presence of the auxiliary binder for the analyte, thereby significantly increasing the sensitivity of the assay.

The present invention also provides improved reagent systems and test kits for carrying out the binding assay method.

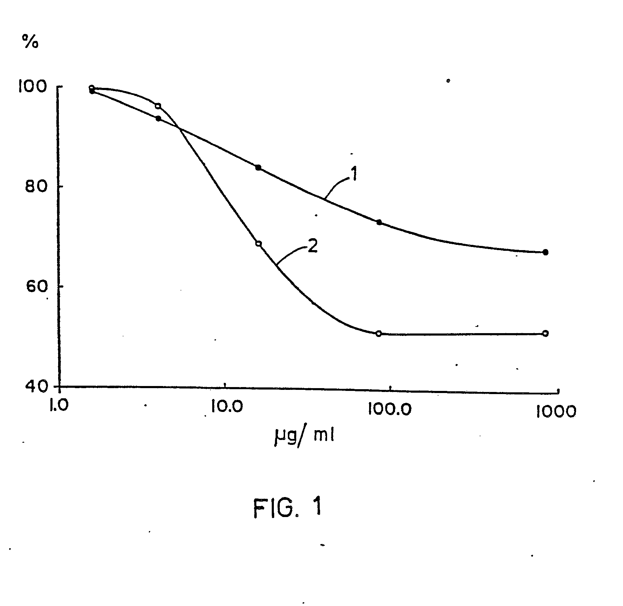

Brief description of the drawing

The drawing is a graphical representation of data presented in the examples which follow demonstrating the enhanced signal modulation by the addition of the auxiliary binder, which in the example used is unlabeled anti-analyte.

Description of the preferred embodiments

In the context of this disclosure, the following terms shall be defined as follows unless otherwise indicated:

Analyte—the compound, or class of related compounds, whose presence or amount in a test sample is under determination.

Binding partner of the analyte—any substance, or class of substances, which has a specific binding affinity for the analyte.

Homogeneous binding assay—an assay based on the specific binding between analyte and its binding partner in which the label response is measured without physical separation of the bound-labeled species from the free-labeled species.

Reagent system—a composition, test device or apparatus, test kit, or other physical arrangement, means, or combination of reagents for use in performing the present assay.

Analyte

The present invention can be applied to the assay of any analyte for which a binding partner is available or can be prepared. In most cases, the analyte is a polypeptide, protein, carbohydrate, glycoprotein, or other organic molecule of sufficient size or allow the binding of both the labeled binding partner and the auxiliary binder to a single analyte molecule. Usually, the analyte is an immunologically active polypeptide or protein, usually having a molecular weight of between about 10,000 and about 10,000,000, such as an antibody or antigenic polypeptide or protein. The present invention is particularly advantageous as applied to the determination of relatively high molecular weight materials in the classes of polypeptides, proteins, polysaccharides, polynucleic acids and the like. A particular advantage is gained where the analyte is an especially scarce material as is the case with many high molecular weight constituents of test samples of analytical interest, e.g., biological samples such as serum, plasma, whole blood, urine, and saliva. The analyte will be polyvalent, that is, able to bind both the labeled binding partner and the auxiliary binder, and will usually have a molecular weight greater than about 10,000, and of ten times greater than about 100,000.

Representative polypeptide analytes are angiotensin I and II, C-peptide, oxytocin, vasopressin, neurophysin, gastrin, secretin, bradykinin, and glucagon.

Representative protein analytes include the classes of protamines, mucoproteins, glycoproteins, globulins, alumins, scleroproteins, phosphoproteins, histones, lipoproteins, chromo-proteins, and nucleoproteins. Examples of specific proteins are prealbumin, $\alpha_1$-lipoprotein, human serum albumin, $\alpha_1$-glycoprotein, trans-cortin, thyroxine binding globulin, haptoglobin, hemoglobin, myoglobin, ceruloplasmin, $\alpha_2$-lipo-protein, $\alpha_2$-macroglobulin, $\beta$-lipoprotein, erythro-poietin, transferin, hemopexin, fibrinogen, the immunoglobulins such as IgG, IgM, IgA, IgD, and IgE, and their fragments, e.g., $F_c$ and $F_{ab}$, comple-ment factors, prolactin, blood clotting factors such as fibrinogen and thrombin, insulin, melano-tropin, somatotropin, thyrotropin, follicle stimulating hormone, luteinizing hormone, chorionic gonadotropin, thyroid stimulating hormone, placental lactogen, intrinsic factor, transcobalamin, serum enzymes such as alkaline phosphatase, lactic dehydrogenase, amylase, lipase, phosphatases, cholinesterase, glutamic oxaloacetic transaminase, glutamic pyruvic transaminase, and uropepsin, endorphins, enke-phalins, protamine, tissue antigens, bacterial antigens, and viral antigens such as hepatits associated antigens (e.g., $HB_sAg$, $HB_cAg$ and $HB_eAg$).

Labeled binding partner

This component is a conjugate of a binding partner for the analyte and a seric-sensitive label.

The binding partner component can be selected from materials which have a specific binding affinity for the analyte. In most cases, the binding partner will be a macromolecule which binds to the analyte in noncovalent, essentially reversible manner. Involved in such binding between analyte and the binding partner will be hydrogen and ionic bonding, van der Waals forces, and dipole-dipole interactions. Other bonding forces may also be involved, including in certain instances covalent bonding. The analyte and binding partner form specific binding pairs, with the analyte being either one of the pair and the binding partner being the other. Such binding pairs include the following groups: antigens/haptens and antibodies thereto; lectins and carbohydrates; complementary polynucleic acids, e.g., RNA and DNA; hormones, vitamins, metabolites and drugs, and their binding counterparts or receptors, usually binding proteins; enzymes and their interactive components, e.g., substrates, coenzymes, and inhibitors.

Most commonly, the binding partner will be an anti-analyte, i.e., an immunologically-derived binding substance specific or selective for binding the analyte. Thus, the anti-analyte can comprise whole antibodies raised against the analyte, or fragments or aggregates of such antibodies. When in the form of whole antibody, anti-analyte can belong to any of the classes and subclasses of known immunoglobulins, e.g., IgG, IgM, IgE, and so forth. Any fragment of any such antibody which retains specific binding affinity for analyte can also be employed, for instance, the fragments of IgG conventionally known as Fab, Fab', and $F(ab')_2$. In addition, aggregates, polymers, and conjugates of immunoglobulins or their fragments can be used where appropriate. Such poly(anti-analyte) can be prepared in any available manner so as to maintain binding affinity for analyte. Other forms of anti-analyte can be employed so long as the material selected has a specific binding affinity for the analyte concerned.

The immunoglobulin source for the anti-analyte can be obtained in any available manner. Usually, anti-analyte immunoglobulin will be obtained by conventional antiserum techniques or monoclonal techniques. Antiserum containing anti-analyte is obtained by well-established techniques involving immunization of an animal, such as a rabbit, guinea pig, or goat, with an appropriate immunogen. State-of-the-art reviews are provided by Parker, *Radioimmunoassay of Biologically Active Compounds*. Prentice-Hall (Englewood Cliffs, New Jersey, U.S.A., 1976), Butler, *J. Immunol. Meth.* 7:1-24 (1975); Weinryb and Shroff, *Drug Metab. Rev.* 10:271—283 (1975); Broughton and Strong, *Clin. Chem.* 22:726—732 (1976); and Playfair et al, *Br. Med. Bull.* 30:24—31 (1974).

Monoclonal anti-analyte is preferably used as the binding partner in the labeled conjugate of the present invention for the reasons given in the aforementioned EP—A—0088974. Monoclonal antibodies are chemically homogeneous and are obtained by somatic cell hydridization techniques; see *Lymphocyte Hydridomas*, ed. Melchers et al, Springer-Verlag (New York 1978) and *Methods in Enzymology 73 (Part B)*; 3—46 (1981). In general, monoclonal antianalyte immunoglobulin is produced by fusing lymphocytes which produce such antibody with myeloma cells to form hydridomas, isolating a hydridoma clone which secretes the desired antibody, the harvesting the secreted monoclonal antibody. The lymphocytes involved in hydridization are commonly spleen cells removed from an animal such as a mouse or a rat which has been immunized in the conventional manner against the analyte.

The label to which the binding partner is conjugated or linked can be any substance which interacts with another molecule or molecules (a member of the reagent detection system) to give a detectable response, where such interaction is sterically sensitive, that is where binding of analyte to the labeled binding partner sterically hinders the response-producing interaction. The mass of the labeled conjugate will be selected to be significantly increased upon binding of the analyte. Generally, as the mass of the analyte becomes relatively larger, the steric effects in the environment of the label become more pronounced.

The label itself will preferably be small, e.g., of molecular weight less than 50,000, generally less than 10,000, more usually less than 4,000, and preferably less than 2,000, and the detection system member or members with which it interacts will preferably be significantly larger, e.g., more than 5 times larger, more usually 10 times larger, and preferably 20 to 100 times or more larger than the label. Accordingly, in the most preferable systems with labels having masses of the order of 100 to 2,000 daltons, at least one member of the detection system with which the label must interact to provide the detectable signal will be of the order of 10,000 to 200,000 daltons or greater. Such size relationship between the label and the detection system member increases the probability of a significant steric effect upon binding of analyte with the labeled binding partner. Preferred labels therefore are participants in an enzyme-catalyzed reaction, such as enzyme substrates, coenzymes, enzyme prosthetic groups, and enzyme inhibitors, since a wide variety of enzymic reactions are available from which to choose assay components. Many small substrates, coenzymes, and inhibitors are known for enzymes of sufficiently large molecular weight to have the preferred size relationship between label and its interacting detection system member. This applies likewise for prosthetic groups and their corresponding apoenzymes. Similarly, many enzymes are known which have significantly larger substrates or for which artificially large substrates, coenzymes, or inhibitors can be prepared by coupling small substrates, coenzymes, and inhibitors to high molecular weight backbone materials such as water soluble polymers.

Specifically preferred labels are those described in detail in the aforementioned EP—A—0088974, and briefly include enzyme substrate and coenzyme labels, U.S. Pat. No. 4,279,992 and U.K. Pat. Spec. 1,552,607; enzyme prosthetic group labels, U.S. Pat. No. 4,238,565; enzyme modulator (e.g., inhibitor) labels, U.S. Pat. Nos. 4,134,792 and 4,273,866; enzyme labels, U.S. Patent Nos. 3,817,837 and 4,043,872; chemically excited fluorescent labels, U.S. Pat. No. 4,238,195; and epitope labels, U.S. Pat. Nos. 3,935,074 and 3,998,943.

The selected label is chemically joined to the binding partner to form the labeled binding partner component of the present invention. A wide variety of means for linking the two elements are available and known in the art. The linkage can involve a chemical bond or a chain comprising from 1 to 50 atoms, more commonly 1 to 30 atoms, and usually 1 to 20 atoms, excluding hydrogen, principally composed of carbon and heteroatoms selected from nitrogen, oxygen, phosphorous, and sulfur. Conventional linking groups are described at length in the literature. See for example U.S. Pat. Nos. 4,230,797; 4,279,992; 3,817,837; 3,935,074; and 3,996,345. The linking group can be comprised of a side arm group put on the label for the purposes of spacing and/or functionalizing the label for coupling to the binding partner and/or the residue from a bifunctional coupling agent used in linking the label or derivatized label to the binding partner.

The steric effects of the binding of analyte and the binding partner are also enhanced by selecting binding partners having masses that will be significantly increased by complexation with analyte. In general, the binding partner preferably will be smaller than 10 times the mass of the analyte, more usually smaller than the absolute mass of the analyte, and more preferably smaller than 0.25 the mass of the analyte. Where anti-analyte is used, the mass of the binding partner can be decreased by selecting antibodies of an immunoglobulin class which have low molecular weights, e.g., IgG antibodies have molecular weights around 150,000 whereas those of the IgM class have molecular weights around 900,000. Also, one can selectively cleave antibodies to give fragments of lower molecular weight which retain their specific binding affinity for analyte, e.g., various fragments of IgG antibodies can be prepared such as Fab (50,000 daltons), Fab' (53,000 daltons), and F(ab')$_2$ (106,000 daltons).

Auxiliary binder

Any macromolecule which selectively binds the analyte can be used as the auxiliary binder in the present invention. By macromolecule is intended a polymeric molecule of natural or synthetic origin having a molecular weight greater than about 5000, usually greater than 10,000, preferably greater than about 40,000, and often greater than 100,000. By selective binding is intended that the auxiliary binder will bind significantly with the analyte, and perhaps other materials present in the reaction mixture, but not labeled binding partner. Normally, the auxiliary binder will be an unlabeled binding partner for the analyte as defined above, and therefore, in the usual case will be a binding protein e.g., antibody, lectin, receptor protein, and the like. Preferably, the auxiliary binder will be an anti-analyte, e.g, an antibody or a fragment or aggregate thereof. When used as the auxiliary binder, anti-analyte may be polyclonal (obtained by conventional antiserum techniques) or monoclonal.

Of course, the auxiliary binder may be a binding protein other than anti-analyte in appropriate circumstances. For instance, the analyte may be a particular carbohydrate or a class of carbohydrates and one can use a labeled antibody as the labeled binding partner and a lectin as the auxiliary binder. In another case, the analyte may be IgG and one can use a labeled antibody other than of the IgG class or a labeled IgG fragment and as auxiliary binder is used protein A (a protein known in the literature to bind to the Fc portion of IgG). Other situations in which various nonantibody binding proteins can be used as the auxiliary binder will be evident to one working in the field.

It is preferred that the auxiliary binder and the labeled binding partner compete minimally for binding to analyte. In the most preferred embodiment, analyte can freely accommodate binding of both labeled binding partner and auxiliary binder in order that substantially all analyte/labeled binding partner complexes that are formed in the assay system become bound by the auxiliary binder to enhance steric hindrance of access of the detection system member which interacts with the label. Therefore, the auxiliary binder and the binding partner which is labeled are preferably not identical. Where the binding partner is monoclonal anti-analyte as is preferred and the auxiliary binder is polyclonal anti-analyte, the test sample is preferably first contacted with the labeled monoclonal anti-analyte for a period of time and then the unlabeled polyclonal anti-analyte added. In this way, competition between the labeled binding partner and the auxiliary binder for binding to the analyte is minimized. Where both the binding partner and the auxiliary binder are monoclonal anti-analyte, they are preferably selected to bind to different determinant sites on the analyte and may be added simultaneously.

Auxiliary binders other than naturally occurring binding proteins can be used provided that they do not bind substantially to the labeled binding partner to avoid modulation of the label response due to steric effects unrelated to the presence of analyte. For example, where the analyte itself is a binding protein, the labeled binding partner can be an antibody to the binding protein analyte and the auxiliary binder can be a corresponding macromolecular material which the binding protein analyte binds, e.g., the material which it binds in nature if such material is macromolecular in size or the material in a form that renders it macromolecular such as a polymerized form or as the material coupled to a macromolecular support or polymer. Such a situation is presented where the analyte is thyroxine binding globulin (TBG). In the assay one would use labeled anti-TBG and, as the auxiliary binder, thyroxine (T-4) or triiodothyronine (T-3) coupled to a polymer support. Another such situation is an assay for the binding protein avidin. Here, one would use labeled anti-avidin and, as the auxiliary binder, a biotin-polymer conjugate. Yet another example is the assay for the enzyme dihydrofolate reductase (DFR) .in which labeled anti-DFR is used with a methotrexate-polymer conjugate as the auxiliary binder.

In addition, the present invention is applicable to the assay of analytes other than binding proteins using auxiliary binders which likewise are not binding proteins. For instance, a lectin can be the analyte with labeled anti-lectin being the labeled binding partner and a carbohydrate which binds to the lectin being the auxiliary binder. Also, a histone can be the analyte with labeled anti-histone being the labeled binding partner and a polynucleic acid, e.g., DNA, which binds the histone being the auxiliary binder. Even further, concanavalin A can be determined using a labeled antibody and, as the auxiliary binder, dextran. One could also assay for a clotting factor (IIa, IXa, Xa, XIa, XIIa, or Kallikrein) using a labeled form of the plasma serum protease inhibitor conventionally referred to as anti-thrombin III and, as the auxiliary binder, heparin (a sulfated proteoglycan of molecular weight around 10,000).

The auxiliary binder need not be specific for the analyte, that is, it can bind to other materials in the reaction mixture, provided that it does not bind significantly to labeled binding partner or otherwise nonspecifically modulate the label response.

Reaction mixture and conditions

The test sample to be assayed can be a naturally occurring or artificially formed liquid suspected to contain the analyte, and usually is a biological fluid or a dilution thereof. Biological fluids that can be assayed include serum, plasma, urine, saliva, milk, and amniotic and cerebrospinal fluids.

The binding reaction will in almost all cases be allowed to proceeds under mild conditions. The reaction mixture will be in general an aqueous medium with any desirable organic cosolvents being present in minor amounts. The temperature of the reaction will be maintained at a constant level in normal circumstances throughout the incubation period and the measurement step. Temperatures will generally be between 5 and 50°C, more usually between 20 and 40°C. Preferably, the reaction will proceed at room temperature. The pH of the reaction mixture will vary between 5 and 10, more usually between 6 and 9.

The concentration of various reagents will depend on the level of analyte expected in the test medium, with such level usually being between $10^{-3}$ and $10^{-12}$M. As in the case of the previously described reaction parameters, selection is primarily based on empirically derived optimization balanced against the preferences and needs of the technician who will ultimately perform assays on a routine basis. None of the parameters therefore is of a critical nature to the present invention, rather they are all within the ordinary skill in the art.

Reagent system

The reagent system, i.e., reagent combination or means, of the present invention comprises all of the essential chemical elements required to conduct a desired assay method encompassed by the present invention. The reagent system is presented in a commercially packaged form, as a composition or admixture where the compatibility of the reagents will allow, in a test device configuration, or as a test kit, i.e., a packaged combination of one or more containers holding the necessary reagents. The reagent system comprises the reagents appropriate for the binding reaction system desired including the labeled binding partner, the auxiliary binder, and any necessary reagents for the label detection system, all as defined hereinbefore. Such binding reaction reagents can include in addition any other optional reagents for performing the particular assay technique involved. Of course, the reagent system can include other reagents as are known in the art and which may be desirable from a commercial and user standpoint, such as buffers, diluents, standards, and so forth. A particularly preferred test kit comprises (1) a labeled monoclonal anti-analyte preparation in which the monoclonal anti-analyte is substantially the only labeled component, (2) an appropriate reagent detection system for the label employed as described herein, and (3) unlabeled anti-analyte as the auxiliary binder. Also preferred is a test device comprising the reagent system and a solid carrier member incorporated therewith. The various forms of such test device are described in EP—A—0051213.

The present invention will now be illustrated, but is not intended to be limited, by the following example.

Examples
Determination of human IgG

A. Purification of monoclonal antibody to human IgG

Ascites fluid [2.5 milliliters (ml)] containing Fc-specific monoclonal antibody to human IgG (Bethesda Research Laboratories, Inc., Gaitherss-burg, Maryland USA), was dialyzed against 4 liters (l) of 5 millimolar (mM) sodium phosphate buffer, pH 8.1, until the conductivity of the dialyzed material approximated that of the 5 mM phosphate buffer.

The dialyzed ascites fluid was applied to a column of Blue Sepharose® (Pharmacia Fine Chemicals AB, Uppsala, Sweden) (1 cm×9 cm) that was equilibrated in 5 mM phosphate buffer, pH 8.1. Mouse serum albumin was retained on the column, but other proteins, including the monoclonal antibody, passed through the column. These fractions were pooled and applied directly to a DEAE-cellulose column (Whatman, Inc., Clifton, New Jersey USA) (1 cm×18.5 cm) that had been washed with 200 mM sodium phosphate buffer, pH 8.1, and then equilibrated with 5 mM sodium phosphate buffer, pH 8.1. The column was eluted with a linear gradient of sodium phosphate, pH 8.1, from 5 mM to 100 mM (200 ml total gradient). Essentially pure monoclonal antibody eluted at ~40 mM sodium phosphate. The fractions were pooled (~85% recovery) and this preparation was used for labeling.

B. Labeling of purified monoclonal antibody

An aqueous solution of flavin $N^6$-(6-aminohexyl) adenine dinucleotide (0.9 ml, 2.4 mM, prepared as described in U.S. Patent No. 4,255,566) was taken to dryness on a rotary evaporator. The following reagents were added in the order given: 0.3 ml of 0.5 M sodium carbonate, pH 10.0; 39 microliters (µl) of triethylamine; and 33.8 mg [138 micromoles (µmol)] of dimethyladipimidate dihydrochloride (Pierce Chemical Co., Rockford, Illinois USA).

The reaction mixture was incubated at 37°C for five minutes. The reaction was terminated by passing the reaction mixture over a column of Sephadex® G-10 (Pharmacia Fine Chemicals AB, Uppsala, Sweden) (1 cm×10.5 cm) that was equilibrated with 20 mM sodium carbonate, pH 10.0. The first 1.4 ml of yellow effluent were collected and evaporated nearly to dryness on the rotary evaporator.

Sodium carbonate buffer (1M, pH 10.0, 100 µl) was added to the residue, followed by 1.0 ml of purified monoclonal antibody preparation from Part A above 1.5 mg/ml, 9.4 nmol). The reaction mixture was incubated at 37°C for twenty minutes. The reaction was terminated and the unreacted $N^6$-aminohexyl-FAD removed by passing the reaction mixture over a column of Sephadex® G-25 (medium) (1.3 cm×48 cm) equilibrated and eluted with 0.1 M sodium phosphate, pH 7.0. Fractions (2 ml) were collected and their absorbance was monitored at 280 nm and 450 nm. The first peak of eluted material with significant absorbance at these wavelengths contained the labeled monoclonal antibody. The label density was 4.6 moles of $N^6$-aminohexyl-FAD per mole of antibody.

C. Homogeneous immunoassay for human IgG

The effect of unlabeled polyclonal anti-(human IgG) on an FAD-based Apoenzyme Reactivation Immunoassay System ("ARIS"; see U.S. Pat. No. 4,238,565) for determining human IgG using FAD-labeled monoclonal anti-(human IgG) was studied as follows.

Each cuvette in a series received 1.9 ml of a reagent containing 92 mM sodium phosphate buffer, pH 7.0, 1% (W/V) bovine serum albumin, 2 mM 3,5-dichloro-2-hydroxbenzene sulfonate, 21 µg/ml horseradish peroxidase and 0.105 M glucose. Next, each cuvette received 25 µl of labeled monoclonal antibody from Part B above (8.6 mM FAD) in 0.1 M sodium phosphate buffer, pH 7.0, containing 0.1% bovine serum albumin. Then each cuvette received 16 µl of goat antibody to glucose oxidase. Aliquots (25 µl) of 0.1 M sodium phosphate buffer, pH 7.0, 0.1% bovine serum albumin, containing various levels of human IgG were added to the appropriate cuvettes. The reaction mixtures were incubated at 25°C for 30 minutes and then an aliquot (2 or 10 µl) or unlabeled polyclonal antibody to human IgG (Accurate Chemical and Scientific Corp., Hicksville, New York USA) was added to the appropriate cuvettes. The reaction mixtures were incubated at 25°C for an additional 30 minutes and then 50 µl of apoglucose oxidase (8 µM FAD binding sites, see U.S. Pat. No. 4,268,631) in 0.1 M sodium phosphate buffer, pH 7.0, containing 50% glycerol, 8 mM 4-amino-antipyrine and 0.02% (W/V) sodium azide. The complete reaction mixtures were incubated at 25°C for 30 minutes and then their absorbances at 520 nm were recorded.

The results are shown in the table below.

| Human IgG (µg/ml in 25 µl Aliquot) | Aliquot of Unlabeled Polyclonal Antibody (µl) | | |
|---|---|---|---|
| | 0 | 2 | 10 |
| | ($A_{520nm}$ after 30 minutes) | | |
| 0 | 1.156(100%) | 1.172(100%) | 1.117(100%) |
| 1.67 | 1.146(99.0%) | 1.110(94.7%) | 1.112(99.6%) |
| 4.0 | 1.079(93.3%) | 1.008(86.0%) | 1.074(96.1%) |
| 16.6 | 0.970(83.9%) | 0.731(62.8%) | 0.768(68.8%) |
| 83 | 0.846(73.1%) | 0.530(45.2%) | 0.569(50.9%) |
| 830 | 0.787(68.0%) | 0.686(58.5%) | 0.578(51.7%) |

The data show that in the absence of unlabeled polyclonal anti-(human IgG), as the amount of human IgG present increased the amount of color generated by recombination of FAD in the FAD-labeled monoclonal anti-(human IgG) with apoglucose oxidase decreased. When unlabeled polyclonal anti-(human IgG) was present, color generation also varied inversely with the level of human IgG present. However, whereas in the absence of unlabeled antibody color generation was inhibited to about 70% of that in the absence of analyte at the highest human IgG concentration, in the presence of 10 µl of unlabeled antibody color generation was inhibited to 50% compared to the zero level concentration for the analyte.

This effect is dramatically illustrated by the graphical representation in the drawing comparing the inhibition curves in the absence of unlabeled antibody and in the presence of 10 µl of unlabeled antibody. The increased inhibition of the label response in the presence of the auxiliary binder provides a significantly more sensitive assay.

## Claims

1. A homogeneous binding assay method for determining a polyvalent analyte in a test sample, wherein said sample is combined with labeled anti-analyte and a reagent detection system for the label comprised in said labeled anti-analyte, said label interacting with a member of said detection system to provide a response which is measurably different when said labeled anti-analyte is bound to said analyte compared to when not so bound due to steric hindrance of access of said detection system member to said label; and wherein said response is measured as a function of the analyte in the test sample, characterized in that said test sample is combined additionally with a macromolecular binder which binds with said analyte but not significantly with said labeled anti-analyte.

2. The method of Claim 1 characterized in that said macromolecular binder is a binding protein.

3. The method of Claim 2 characterized in that said binding protein is an antibody or an aggregate or fragment thereof.

4. The method of any of Claims 1 to 3, characterized in that said labeled anti-analyte is a labeled form of a monoclonal antibody or of an aggregate or fragment thereof.

5. The method of any one of Claims 1 to 4, characterized in that said label is a participant in an enzyme catalyzed reaction comprised in said detection system and preferably is an enzyme substrate, a coenzyme, an enzyme prosthetic group, an enzyme inhibitor, or an enzyme.

6. The method of any of Claims 1 to 5, characterized in that said analyte has a molecular weight greater than 10.000, preferably greater than 100.000.

7. A homogeneous immunoassay method according to Claim 1 for determining a polyvalent analyte having a molecular weight greater than 10.000 in a test sample, wherein said sample is combined with (i) a labeled monoclonal anti-analyte preparation in which said monoclonal anti-analyte is substantially the only labeled component, and (ii) a reagent detection system for the label comprised in said labeled monoclonal anti-analyte, said label interacting with a member of said detection system to provide a response which is measurably different when said labeled anti-analyte is bound to said analyte compared to when not so bound due to steric hindrance of access of said detection system member to said label; and wherein said response is measured as a function of the analyte in the test sample, characterized in that said test sample is combined additionally with unlabeled anti-analyte.

8. A reagent system for performing a homogeneous binding assay to determine a polyvalent analyte in a test sample, comprising:

(1) labeled anti-analyte for said polyvalent analyte;

(2) a reagent detection system for the label comprised in said labeled anti-analyte, said label interacting with a member of said detection system to provide a response which is measurably different when said labeled anti-analyte is bound to said polyvalent analyte compared to when not so bound due to steric hindrance of access of said detection system member to said label; characterized in that it additionally comprises

(3) a macromolecular binder which binds with said polyvalent analyte but not significantly with said labeled anti-analyte.

9. A test kit for performing a homogeneous immunoassay according to claim 1 to determine a polyvalent analyte having a molecular weight greater than 10.000 in a test sample, comprising:

(1) a labeled monoclonal anti-analyte preparation in which said monoclonal anti-analyte is substantially the only labeled component,

(2) a reagent detection system for the label comprised in said labeled monoclonal anti-analyte, said label interacting with a member of said detection system to provide a response which is measurably different when said labeled anti-analyte is bound to said analyte compared to when not so bound due to steric hindrance of access of said detection system member to said label; and

(3) unlabeled anti-analyte.

10. A test device for the homogeneous binding assay determination of an analyte in a test sample, characterized in that it comprises the reagent system of Claim 8 and a solid carrier member incorporated therewith.

## Patentansprüche

1. Homogenes Bindungs-Assay-Verfahren zur Bestimmung eines polyvalenten Analyten in einer Testprobe, wobei die genannte Probe mit einem markierten Anti-Analyten und einem Reagenz-Nachweissystem auf den Marker, welcher in dem genannten markierten Anti-Analyten vorliegt, kombiniert wird, und der genannte Marker mit einem Element des Nachweissystems unter Erhalt eines Signals in Wechselwirkung tritt, wobei sich das Signal bei der Messung unterscheidet, je nachdem ob der genannte markierte Anti-Analyt an den Analyten gebunden ist oder aufgrund einer sterischen Zugangsbehinderung des genannten Nachweissystem-Elementes an den Marker nicht gebunden ist; und wobei das genannte Signal als eine Funktion des Analyten in der Testprobe gemessen wird, dadurch gekennzeichnet, dass die genannte Testprobe zusätzlich mit einem makromolekularen Bindemittel versehen wird, welches mit dem genannten Analyten bindet, jedoch mit dem genannten markierten Anti-Analyten keine signifikante Bindung eingeht.

2. Verfahren nach Anspruch 1, dadurch gekennzeichnet, dass das genannte makromolekulare Bindemittel ein Bindungsprotein darstellt.

3. Verfahren nach Anspruch 2, dadurch gekennzeichnet, dass das genannte Bindungsprotein einen Antikörper oder ein Aggregat oder ein Fragment desselben darstellt.

4. Verfahren nach einem oder mehreren der Ansprüche 1 bis 3, dadurch gekennzeichnet, dass der genannte markierte Anti-Analyt in einer markierten Form eines monoklonalen Antikörpers oder eines Aggregates oder Fragmentes desselben vorliegt.

5. Verfahren nach einem oder mehreren der Ansprüche 1 bis 4, dadurch gekennzeichnet, dass der genannte Marker an einer enzymkatalysierten Reaktion, die von dem genannten Nachweissystem umfasst wird, teilnimmt wird vorzugsweise eine Enzymsubstrat, ein Coenzym, eine prosthetische Gruppe eines Enzyms, einen Enzyminhibitor oder ein Enzym darstellt.

6. Verfahren nach einem oder mehreren der Ansprüche 1 bis 5, dadurch gekennzeichnet, dass der genannte Analyt ein Molekulargewicht über 10,000, vorzugweise über 100.000 aufweist.

7. Homongenes Immunoassay-Verfahren nach Anspruch 1, zur Bestimmung eines polyvalenten Analyten mit einem Molekulargewicht von über 10.000 in einer Testprobe, wobei die genannte Probe kombiniert wird mit: (i) einer markierten monoclonalen Anti-Analyt-Präparation, in welcher der genannte monoclonale Anti-Analyt im wesentlichen die einzige markierte Komponente darstellt, und (ii) einem Reaktions-Nachweissystem für den Marker, welcher in dem genannten markierten monoklonalen Anti-Analyten vorliegt, wobei der genannte Marker mit einem Element des Nachweissystems unter Bildung eines Signals in Wechselwirkung tritt, welches sich bei der Messung unterscheidet, je nachdem ob der genannte markierte Anti-Analyt an den genannten Analyten gebunden ist, oder aufgrund einer sterischen Zugangsbehinderung des genannten Nachweissystem-Elementes an den Marker nicht gebunden ist; und das genannte Signal als eine Funktion des Analyten in der Testprobe gemessen wird, dadurch gekennzeichnet, dass die genannte Testprobe zusätzlich mit einem nicht-markierten Anti-Analyten kombiniert wird.

8. Reaktionssystem zur Durchführung eines homogenen Bindungsassays zur Bestimmung eines polyvalenten Analyten in einer Testprobe, gekennzeichnet, durch:
(1) einen marktierten Anti-Analyten für den genannten polyvalenten Analyten;
(2) eine Rektions-Nachweissystem für den Marker, welcher in dem genannten markierten Anti-Analyten vorliegt, wobei der Marker mit einem Element des genannten Nachweissystems unter Bildung eines Signals in Wechselwirkung tritt, und sich das Signal bei der Messung unterscheidet, je nachdem ob der genannte Anti-Analyt an den polyvalenten Analyten gebunden ist oder ob er aufgrund einer sterischen Zugangsbehinderung des genannten Nachweissystem-Elementes an den Marker nich gebunden ist; dadurch gekennzeichnet, dass das Reaktionssystem zusätzlich umfasst:
(3) ein makromolekulares Bindemittel, welches an den genannten polyvalenten Analyten bindet, jedoch mit dem genannten markierten Anti-Anälyten keine signifikante Bindung eingeht.

9. Testkit zur Durchführung eines homogenen Immunoassays nach Anspruch 1, zur Bestimmung eines polyvalenten Analyten mit einem Molekulargewicht von über 10.000 in einer Testprobe, gekennzeichnet, durch:
(1) eine markierte monoclonale Anti-Analyt-Präparation, in welcher der genannte monoclonale Anti-Analyt im wesentlichen die einzige markierte Komponente darstellt,
(2) ein Reagenz-Nachweissystem für den Marker, der in dem genannten markierten monoclonalen Anti-Analyten vorliegt, wobei der genannte Marker mit einem Element des genannten Nachweissystems unter Bildung eines Signals in Wechselwirkung tritt, wobei sich das Signal bei der Messung unterscheidet, je nach dem ob der markierte Anti-Analyt an den genannten Analyten gebunden ist, oder ob er aufgrund einer sterischen Zugangsbehinderung des genannten Nachweissystem-Elementes an den Marker nicht so gebunden ist; und
(3) einen nicht-markierten Anti-Analyten.

10. Testvorrichtung für eine homogene Bindungs-Assay-Bestimmung eines Analyten in einer Testprobe, dadurch gekennzeichnet, dass sie das Reagenz-System von Anspruch 8 und einen festen Träger, der mit demselben inkorporiert ist, umfasst.

## Revendications

1. Méthode d'analyse par liaison homogène pour le dosage d'un analyte polyvalent dans un échantillon à analyser, dans laquelle ledit échantillon est mis en présence d'un anti-analyte marqué et d'un système de détection à réactifs pour le marqueur inclus dans ledit anti-analyte marqué, ledit marqueur agissant mutuellement avec un composant dudit système de détection pour engendrer une réponse qui diffère de façon mesurable lorsque ledit anti-analyte marqué est lié audit analyte comparativement au cas où il n'est pas lié de la sorte, par suite de l'empêchement stérique de l'accès dudit composant du système de détection audit marqueur; et ladite réponse est mesurée en fonction de l'analyte dans l'échantillon à analyser, caractérisée en ce que ledit échantillon à analyser est en outre mis en présence d'un liant macromoléculaire qui se lie audit analyte, mais qui ne se lie pas notablement audit anti-analyte marqué.

2. Méthode suivant la revendication 1, caractérisée en ce que ledit liant macromoléculaire est une protéine de liaison.

3. Méthode suivant la revendication 2, caractérisée en ce que ladite protéine de liaison est un anti-corps ou un groupement ou un fragment d'anticorps.

4. Méthode suivant l'une quelconque des revendications 1 à 3, caractérisée en ce que ledit anti-analyte marqué est une forme marquée d'un anticorps monoclonal ou d'un groupement ou d'un fragment d'un tel anticorps.

5. Méthode suivant l'une quelconque des revendications 1 à 4, caractérisée en ce que ledit marqueur est un corps qui participe à une réaction catalysée par un enzyme inclus dans ledit système de détection et est de préférence un substrat d'enzyme, un coenzyme, un groupe prosthétique d'enzyme, un inhibiteur d'enzyme ou un enzyme.

6. Méthode suivant l'une quelconque des revendications 1 à 5, caractérisée en ce que ledit analyte a un poids moléculaire supérieur à 10 000, de préférence supérieur à 100 000.

7. Méthode d'analyse immunologique homogène suivant la revendication 1, pour le dosage d'un analyte polyvalent de poids moléculaire supérieur à 10 000 dans un échantillon à analyser, dans laquelle ledit échantillon est mis en présence (i) d'un préparation d'anti-analyte monoclonal marqué dans laquelle ledit anti-analyte monoclonal constitue pratiquement le seul composant marqué et (2) d'un système de détection à réactifs pour l'indicateur inclus dans ledit anti-analyte monoclonal marqué, ledit indicateur agissant mutuellement avec un composant dudit système de détection pour engendrer une réponse qui diffère de façon mesurable lorsque ledit anti-analyte marqué est lié audit analyte comparativement au cas où il n'est pas lié de la sorte à cause de l'empêchement stérique de l'accès dudit composant du système de détection au marqueur; et ladite réponse est mesurée en fonction de l'analyte dans l'échantillon à analyser, caractérisée en ce que ledit échantillon à analyser est en outre mis en présence d'un anti-analyte non marqué.

8. Système de réactifs pour l'exécution d'une analyse par liaison homogène pour le dosage d'une analyte polyvalent dans un échantillon à analyser, comprenant

(1) un anti-analyte marqué pour ledit analyte polyvalent;

(2) un système de détection à réactifs pour l'indicateur inclus dans ledit anti-analyte marqué, ledit indicateur agissant mutuellement avec un composant dudit système de détection pour engendrer une réponse qui diffère de façon mesurable lorsque ledit anti-analyte marqué est lié audit analyte polyvalent comparativement au cas où il n'est pas lié de la sorte à cause de l'encombrement stérique de l'accès dudit composant du système de détection audit marqueur;

caractérisé en ce qu'il comprend en outre

(3) un liant macromoléculaire qui se lie audit analyte polyvalent mais qui ne se lie pas notablement audit anti-analyte marqué.

9. Kit analytique pour l'exécution d'une analyse immunologique homogène suivant la revendication 1, pour détecter un analyte polyvalent de poids moléculaire supérieur à 10 000 dans un échantillon à analyser, comprenant:

(1) une préparation d'anti-analyte monoclonal marqué dans laquelle ledit anti-analyte monoclonal constitue pratiquement le seul composant marqué;

(2) un système de détection du marqueur inclus dans ledit anti-analyte monoclonal marqué, ledit marqueur agissant mutuellement avec un composant du système de détection pour engendrer une réponse qui diffère de façon mesurable lorsque ledit anti-analyte marqué est lié audit analyte comparativement au cas où il n'est pas lié de la sorte à cause de l'empêchement stérique de l'accès dudit système de détection audit marqueur; et

(3) un anti-analyte non marqué.

10. Dispositif analytique pour la détection par liaison homogène d'un analyte dans un échantillon d'essai, caractérisé en ce qu'il comprend le système de réacfits de la revendication 8 et un support solide qui y est incorporé.

FIG. 1